# EUROPEAN PATENT SPECIFICATION

(11) **EP 1 409 497 B1**
(45) Date of publication and mention of the grant of the patent: **19.01.2005**
(21) Application number: 02764561.3
(22) Date of filing: 12.07.2002
(51) Int. Cl.: C07H 1/00

(54) **METHOD FOR PREPARATION OF LNA PHOSPHORAMIDITES**
VERFAHREN ZUR HERSTELLUNG DES LNA PHOSPHORAMIDITE
ELABORATION DE PHOSPHORAMIDITES D'ACIDE NUCLEIQUE VERROUILLE

(30) Priority: 12.07.2001 DK 200101095
(43) Date of publication of application: 21.04.2004
(73) Proprietor: Santaris Pharma A/S, 2970 Horsholm (DK)
(72) Inventor: KOCH, Troels, 2300 Copenhagen S (DK); ROSENBOHM, Christoph, 3460 Birkerod (DK); PEDERSEN, Daniel, Sejer, Cambridge CB3 OAD (GB)
(74) Representative: Plougmann & Vingtoft A/S
(86) International application number: PCT/DK2002/000488
(87) International publication number: WO 2003/006475

(56) References cited:
- WO-A-98/16540
- SATOSHI OBIKA ET AL: "2'-O,4'-C-methylene bridged nucleic acid (2',4'-BNA): Synthesis and triplex-forming properties I" BIOORGANIC & MEDICINAL CHEMISTRY, vol. 9, 2001, pages 1001-1011, XP002902764
- SATOSHI OBIKA ET AL: "A 2', 4'-Bridged Nucleic Acid containing 2-Pyridone as a Nucleobase: Efficient Recognition of a C-G Interruption by Triplex Formation with a Pyrimidine Motif" ANGEW. CHEM. INT. ED., vol. 40, no. 11, 2001, pages 2079-2081, XP002902765
- SHOJI HAMAMOTO ET AL: "New Approach to the Synthesis of Deoxyribonucleoside Phosphoramidite Derivatives" CHEMISTRY LETTERS, vol. 8, 1986, pages 1401-1404, XP002902766
- HAKANSSON A E ET AL: "CONVENIENT SYNTHESES OF 7-HYDROXY-1-(HYDROXYMETHYL)- 3-(THYMIN-1-YL)-2,5-DIOXABICYCLO2.2.1HEPTA NES: ALPHA-6-RIBO- AND ALPHA-L-XYLO-CONFIGURED LNA NUCLEOSIDES" JOURNAL OF ORGANIC CHEMISTRY, AMERICAN CHEMICAL SOCIETY. EASTON, US, vol. 65, no. 17, 25 August 2000 (2000-08-25), pages 5161-5166, XP000973191 ISSN: 0022-3263

## Description

### FIELD OF THE INVENTION

The present invention relates to a high yielding, fast method for large-scale LNA phosphoramidites synthesis.

### BACKGROUND OF THE INVENTION

Locked Nucleic Acid (LNA) monomers and oligonucleotides were invented in 1997 (W09914226) and have showed promising results as antisense drug candidates. However, there is no production method described in the literature for large scale LNA-phosphoramidite synthesis providing optimal yields of the LNA amidites necessary for efficient preparation of LNA based antisense drugs.

Oligonucleotide synthesis is typically performed using the phosphoramidite method, invented by Caruthers, U54415732, in 1980 and improved a couple of years later by Köster US4725677. LNA oligomers are being synthesised according to the phosphoramidite method, but so far the large scale supply of LNA monomers has been a problem due to slow reactions, side product formation during the reaction and reagents unstable at room temperature have been employed.

The mechanism of phosphoramidite activation and coupling in oligonucleotide syntheses has been studied in detail. Traditionally tetrazole has been used (Dahl *et al.* 1987, *Nucleic Acid Research,* 15, 1729-1743; Beaucage & Iver, 1992, *Tetrahedron,* 48, 2223-2311). The proposed mechanism of tetrazole is two step, first tetrazole protonates trivalent phosphorous followed by displacement of the *N,N*-diisopropylamine by the tetrazolide. This latter intermediate is very reactive with hydroxynucleophiles such as 5'-OH on nucleic acids. Therefore, tetrazole acts both as acid and as nucleophilic agent.

Vargeese *et al.* (Vargeese, C.; Carter, J.; Krivjansky, S.; Settle, A.; Kropp, E.; Peterson, K.; Pieken, W. *Nucleic Acid Research,* 1998, 26, 1046-1050; WO9816540;) have described an activator for the coupling of phosphoramidites to the 5'-hydroxyl group during oligonucleotide synthesis. The activator is 4,5-dicyanoimidazole (DCI) and its effectiveness is thought to be based on its nucleophilicity. It was shown that DCI significantly increased the yield in phosphoramidite oligomerisation.

Vargeese *et al.* has described a method of small scale DNA phosphoramidite thymine monomer synthesis using DCI, but the method they used provided a moderate yield (75%) after several re-precipitations of the amidite. Kittaka *et al.* (Kittaka A., Kuze T.; Amano M.; Tanaka H.; Miyasaka T.; Hirose K.; Yoshida T.; Sarai A.; Yasukawa T. and Ishii S. *Nucleosides & Nucleotides* 18, 2769-2783, 1999) have also used DCI for amidite synthesis but they reported an even lower yield of the product (65%). Kittaka *et al.* (Kittaka A.; Horii C.; Kuze T.; Asakura T.; Ito K.; Nakamura K.T.; Miyasaka T. and Inoue J., *Synthetic letters,* S1, 869-872, 1999) have also made a derivatised uridine phosphoramidite by 3'-*O* phosphitylation using DCI (0.7 eq), but without describing the reaction parameters further. The yield was reported to be 92%. Generally, phosphitylation of uracil and thymine provide the highest yields because these nucleobases usually do not interfere with the phosphitylation reagents.

Accordingly, there is nothing in the literature describing that DCI has been used as an activator for LNA phosphoramidite synthesis.

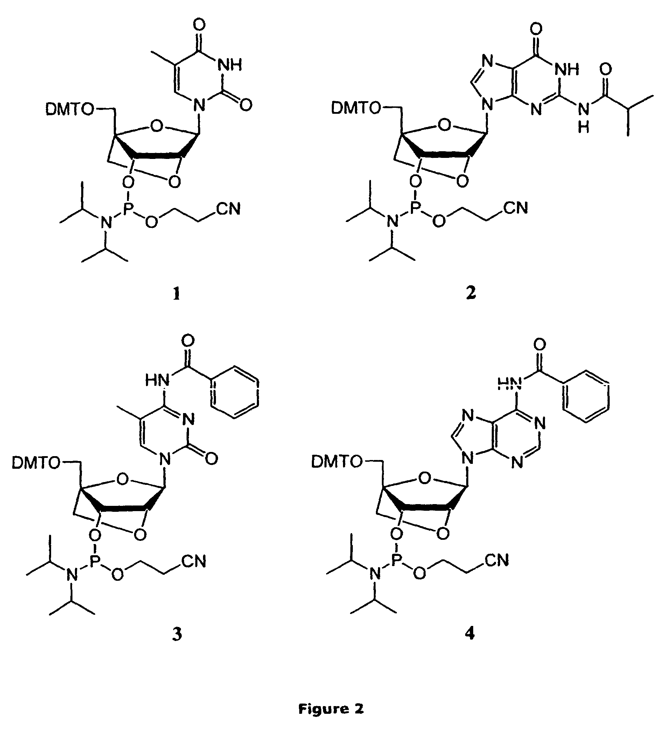

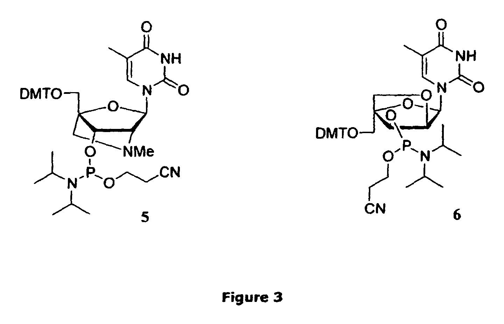

The previously reported oxy-β-D-ribo-LNA (Figure 2) method of synthesis used 2-cyanoethyl-N,N-diisopropylchlorophosphoramidite and diisopropylethylamine (Koshkin, A. A.; Singh, S. K.; Nielsen, P.; Rajwanshi, V. K.; Kumar, R.; Meldgaard, M.; Olsen, C. E.; Wengel, *J*. *Tet.* 1998, 54, 3607-3630). The reported yields were low (LNA-T 70%, LNA-U 58%, LNA-G 64%, LNA-A 73% and no yield for LNA-C was reported). The previously reported method for synthesising the oxy-α-L-ribo thymine phosphoramidite (Figure 3) also utilized 2-cyanoethyl-*N,N*-diisopropylchlorophosphoramidite and diisopropylethylamine and gave a yield of 60% (Hakansson, A.E.; Koshkin, A.A.; Sorensen M.D.; Wengel J. *J.Org.Chem.* 2000, 65, 5161-5166).

Finally Satoshi O. et al (Bioorganic & medicinal chemistry, 9 (2001), 1001-1011) disclose the 2-cyanoethyl-N,N,N',N'-tetraisopropylphosphoramidite in the presence of diisopropylammonium tetrazolide.

### BRIEF DESCRIPTION OF THE FIGURES

Figure 1: Phosphitylation of guanine, a) 2-Cyanoethyl-N,N-diisopropylchlorophosphoramidite, diisopropylethylamine, b) Phosphitylation of guanine 3'OH via the transfer/rearrangement reaction (slow).
Figure 2: Oxy-β-D-ribo-LNA phosphoramidites.
Figure 3: Amine-β-D-ribo-LNA phosphoramidites and oxy-α-L-ribo-LNA thymine phosphoramidite.

### SUMMARY OF THE INVENTION

The present invention provides a novel high yielding and fast method for the large scale synthesis of pure LNA phosphoramidites. In the production of LNA monomers as phosphoramidites, the phosphitylation step is crucial. If necessary also a facile and efficient purification is desired, due to the reactivity of the phosphoramidites, so as to avoid their decomposition during purification. An absolute purity of the amidites is a prerequisite because the monomers are used in long sequential oligomerisations over some time where also impurities can cause the decomposition of the phosphoramidites.

The novel strategy is demonstrated by the synthesis of oxy-β-ribo-LNA phosphoramidites where B is A^{Bz}, ^{Me}C^{Bz}, G^{lbu} and T and oxy α-L-ribo-LNA-T ( Figure 3).

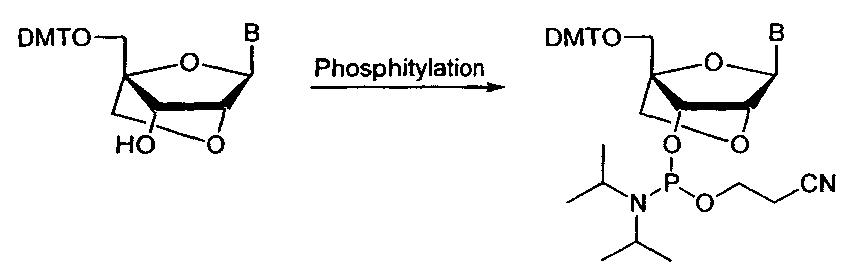

The present invention provides a method for the synthesis of an LNA phosphoramidite, the method comprising phosphitylation of the 3'-OH groups of an LNA monomer with a 2-cyanoethyl-*N*,*N*,*N'*,*N*'-tetra-substituted phosphoramidite in the presence of a nucleophilic activator.

The advantages of the present activation method is:
a) Easy handling
b) All reagents are stabile at room temperature
c) Only 0.7 equivalents DCI are necessary
d) Easy workup
e) Fast and high yielding reaction at room temperature
f) Cost efficient reaction
g) Chromatography not essential for obtaining amidites of high purity

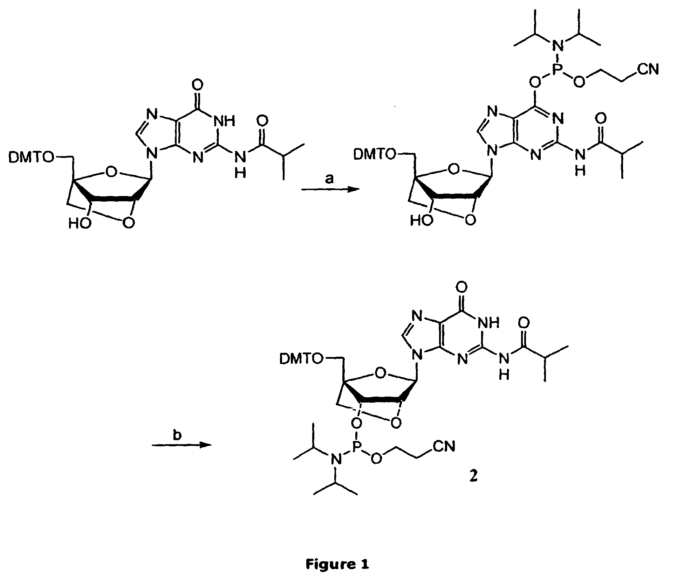

The known phosphitylation side reaction of the guanine nucleobase followed by slow transfer/rearrangement to give the desired amidite (Nielsen, J.; Taagaard, M.; Marugg; J. E.; van Boom, J. H.; Dahl, O. *Nuc.Acid.Res.* 1986, *14*, 7391-7403) (see Figure 1) was not observed using the conditions according to the present invention.

### DETAILED DESCRIPTION OF THE INVENTION

As used herein, the term "LNA monomer" refers to a ribonucleotide having a 2',4'-bridge (in particular a -O-CH₂- (oxy-LNA), -S-CH₂- (thio-LNA), -NR-CH₂- (amino-LNA, R being hydrogen, C₁₋₆-alkyl, phenyl, benzyl, etc.) bridge) as described in the International Patent Application WO9914226 and subsequent WO0056746, WO0056748, WO0066604 and W00228875.

Particularly interesting examples of LNA monomers of are those referred to as oxy-β-D-ribo-LNA (cf. Figure 2), thio-β-D-ribo-LNA, amino-β-D-ribo-LNA, oxy-α-L-ribo-LNA (cf. Figure 3), thio-α-L-ribo-LNA or amino-α-L-ribo-LNA.

As used herein, the term "LNA phosphoramidite" refers to a nucleotide with a protected 5'-hydroxy group (e.g. DMT protected as illustrated in Figures 2 and 3), in which the 3'-hydroxy group is coupled to a trivalent phosphorous atom, which in turn is bonded to a suitable leaving group such as a *N,N*-dialkylamine, e.g. diisopropylamine and a protecting group such as the cyanoethyl (NCCH₂CH₂-) group. Any LNA phosphoramidite that can be used in solid or liquid phase oligonucleotide synthesis can be used in the present invention, including protected dimer and trimer LNA phosphoramidite.

As used herein, the term "2-cyanoethyl-*N,N,N',N'*-tetra-substituted phosphoramidite", also referred to as "PN₂-reagent", refers in short to a 2-cyanoethyl-*N,N,N',N'*-tetra-substituted phosphoramidite, wherein the term "substituted" means a linear, cyclic or branched unsaturated hydrocarbon such as allyl or vinyl or saturated hydrocarbon or substituted hydrocarbon group or aromatic group or substituted aromatic group having 1 to 9 carbon atoms, such as methyl, ethyl, propyl, iso-propyl, pentyl, cyclopentyl, hexyl, cyclohexyl, preferred examples alkyl are methyl, ethyl, propyl, iso-propyl, butyl, tert-butyl, iso-butyl, pentyl, cyclopentyl, hexyl, cyclohexyl, in particular methyl, ethyl, propyl, iso-propyl, tert-butyl, iso-butyl, cyclohexyl, propylene, butylene, pentylene, hexylene, heptylene, where the substituents can be a hetroatom such as oxygen or nitrogen.

The LNA monomer as well as the phosphoramidite LNA monomer may carry any nucleobase in the 1' position.

In the present context, the term "nucleobase" covers naturally occurring nucleobases as well as non-naturally occurring nucleobases. It should be clear to the person skilled in the art that various nucleobases which previously have been considered "non-naturally occurring" have subsequently been found in nature. Thus, "nucleobase" includes not only the known purine and pyrimidine heterocycles, but also heterocyclic analogues and tautomers thereof. Illustrative examples of nucleobases are adenine, guanine, thymine, cytosine, uracil, purine, xanthine, diaminopurine, 8-oxo-*N*⁶-methyladenine, 7-deazaxanthine, 7-deazaguanine, *N*^{*4*}*,N*^{*4*}-ethanocytosin, *N*^{*6*}*,N*^{*6*}-ethano-2,6-diaminopurine, 5-methylcytosine, 5-(*C*³-C⁶)-alkynylcytosine, 5-fluorouracil, 5-bromouracil, pseudoisocytosine, 2-hydroxy-5-methyl-4-triazolopyridin, isocytosine, isoguanin, inosine, *N*^{*6*} alylpurines, *N*⁶-acylpurines, *N*⁶-benzylpurine, *N*⁶-halopurine, *N*⁶-vinylpurine, *N*⁶-acetylenic purine, *N*⁶-acyl purine, *N*⁶-hydroxyalkyl purine, *N*⁶-thioalkyl purine, *N*²-alkylpurines, *N*⁴-alkylpyrimidines, *N*⁴-acylpyrimidines, *N*⁴-benzylpurine, *N*⁴-halopyrimidines, *N*⁴- . vinylpyrimidines, *N*⁴-acetylenic pyrimidines, *N*⁴-acyl pyrimidines, *N*⁴-hydroxyalkyl pyrimidines, *N*⁶-thioalkyl pyrimidines, thymine, cytosine, 6-azapyrimidine, including-6-azacytosine, 2- and/or 4-mercaptopyrimidine, uracil, *C*⁵-alkylpyrimidines, *C*⁵-benzylpyrimidines, *C*⁵-halopyrimidines, *C*⁵-vinylpyrimidine, *C*⁵-acetylenic pyrimidine, *C*⁵-acyl pyrimidine, *C*⁵-hydroxyalkyl purine, *C*⁵-amidopyrimidine, *C*⁵-cyanopyrimidine, *C*⁵-nitropyrimidine, *C*⁵-aminopyrimdine, *N*²-alkylpurines, *N*²-alkyl-6-thiopurines, 5-azacytidinyl, 5-azauracilyl, trazolopyridinyl, imidazolopyridinyl, pyrrolopyrimidinyl, and pyrazolopyrimidinyl. Functional oxygen and nitrogen groups on the base can be protected as necessary or desired. Suitable protecting groups are well known to those skilled in the art, and included trimethylsilyl, dimethylhexylsilyl, t-butyldimenthylsilyl, and *t*-butyldiphenylsilyl, trityl, alkyl groups, acyl groups such as acetyl and propionyl, methanesulfonyl, and p-toluenesulfonyl, imines such as N-dimethylmethyleneeneamine and dibutylmethyleneeneamine. Preferred bases include cytosine, 5-methylcytosine uracil, thymine, adenine and guanine.

LNA phosphoramidites according to the present invention are prepared in high yield with 2-cyanoethyl*-N,N,N',N'*-tetraisopropylphosphoramidite (a PN₂-reagent). This reagent has not successfully been used to prepare LNA phosphoramidites before. Previously 2-cyanoethyl-*N*,*N*-diisopropylchlorophosphoramidite has been used for preparation of LNA amidites. This has not afforted the high purity, high yields and low reaction times according to the present invention.

The surprising ending in the present inventions is that 2-cyanoethyl*-N,N,N',N'*-tetraisopropylphosphoramidite can be activated by 4,5-dicyanoimidazole and that this complex provides the accurate reactivity, during the conditions described, to secure efficient phosphitylation of all 4 LNA DMT-protected nucleosides including the G-nucleoside. With the previous known methods using 2-cyanoethyl*-N,N*-diisopropylchlorophosphoramidite the phosphitylation reaction would proceed via oxygen on the protected guanine nucleobases (Figure 1a) and thereafter be rearranged to the thermodynamically stable 3'-O-amidite (Figure 1b). The disadvantages with the previous method were that the final product contained side products and that it required significantly longer reaction time, (up to 24 h) due to the slow rearrangement resulting in the thermodynamical product.

The traditional procedures for making LNA phosphoramidites provided yields ranging from 14-73%, whereas the present procedure provides yields of 95% or higher. This is also remarkable for the reason that the corresponding reaction for DNA/RNA nucleotides only provides yields of 92%.

Another advantage of the present invention is that the reactions are finished in 0.5-4 hours, preferably 1.0-3.5 hours, and that chromatography of the LNA amidites is unnecessary due to the efficient reaction. Thus, the phosphitylation reaction is typically allowed to proceed for 0.1-12 hours, such as 0.2-8 hours, e.g. 0.5-4.0 hours, such as 1.0-3.5 hours.

An LNA phosphoramidite for direct use in oligonucleotide synthesis may be obtained directly or by simple precipitation of the product from the reaction mixture. Thus, in a particular aspect of the invention, the method comprises at the most one precipitation step.

Suitable nucleophilic activators for use in the present invention include, but are not limited to, 4,5-dicyanoimidazole (DCI), 4-alkylthioimidazole, 2-alkylthioimidazole, 2-nitroimidazole, 4-nitroimidazole, 4,5-dihaloimidazole, 4-haloimidazole, 2-haloimidazole and 5-alkoxytetrazole. DCI is the preferred nucleophilic activator. DCI has a pKₐ of 5.2 and is easily dissolved in acetonitrile.

Suitable PN₂-reagents are for example, but not limited to, 2-cyanoethyl-*N,N,N',N'*-tetraisopropylphosphoramidite and 2-cyanoethyl-*N,N,N',N'*-tetraethylphosphoramidite and the reagents described by Dahl, B.H., Nielsen J. and Dahl O. *Nucleic Acid Research,* 1987, 15, 1729-1743 all incorporated here by reference. It is presently believed that 2-cyanoethyl-*N,N,N',N'*-tetraisopropylphosphoramidite and 2-cyanoethyl-*N,N,N',N'*-tetraethylphosphoramidite are especially relevant, in particular 2-cyanoethyl-*N,N,N',N'*-tetraisopropylphosphoramidite.

A currently preferred combination is where the 2-cyanoethyl-N,N,N',N'-tetra-substituted phosphoramidite is the 2-cyanoethyl-*N,N,N',N'*-tetraisopropylphosphoramidite and the nucleophilic activator is 4,5-dicyanoimidazole.

The molar ratio between the LNA monomer and the nucleophilic activator is typically in the range of 1:0.0001 to 1:10, preferably 1:0.0001 to 1:1, and more preferably 1:0.0001 to 1:0.7.

The molar ratio between the LNA monomer and the PN₂-reagent is typically in the range of 1:0.9 to 1:10, preferably 1:0.95 to 1:5, more preferably 1:1.

The currently most interesting LNA monomers are those which have a nucleobase selected from guanine, thymine, cytosine, 5-methylcytosine, uracil and adenine, typically in the protected from such as N-benzoyl protected cytosine (C^{Bz}), N-benzoyl protected methyl cytosine (^{Me}C^{Bz}), N-iso-butanoyl protected guanine (G^{1bu}), uracil (U), thymine (T), N-benzoyl protected adenine (A^{Bz}).

### EXAMPLES

### EXAMPLE 1 - Phosphitylation methods

Four phosphitylation methods was examined and compared: 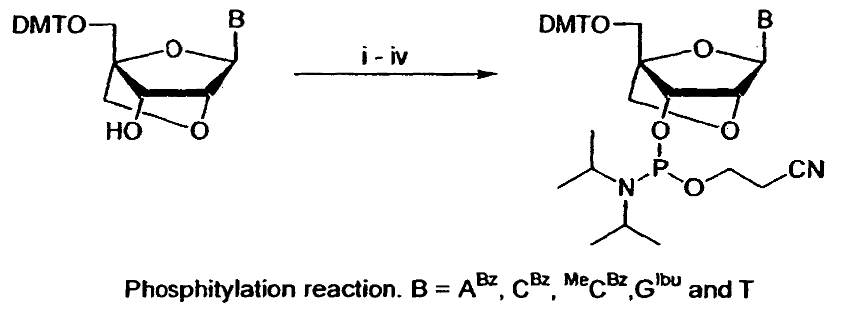
i. 2-Cyanoethyl-*N,N-*diisopropylchlorophosphoramidite, diisopropylethylamine
ii. 2-Cyanoethyl-*N,N,N',N'*-tetraisopropylphosphoramidite, 1*H*-tetrazole
iii. 2-Cyanoethyl-*N,N,N',N'*-tetraisopropylphosphoramidite, pyridinium trifluoroacetate
iv. 2-Cyanoethyl-*N,N,N',N'*-tetraisopropylphosphoramidite, 4,5-dicyanoimidazole

2-Cyanoethyl-*N,N*-diisopropylchlorophosphoramidite appeared to have several disadvantages compared to 2-cyanoethyl-*N,N,N',N'*-tetraisopropylphosphoramidite (PN₂-reagent). It is unstable at room temperature, expensive and difficult to handle due to its high reactivity. Furthermore undesired reactions with the nudeobases are often observed with 2-cyanoethyl-*N,N-*diisopropylchlorophosphoramidite (Figure 1).

Three different activators (1*H*-tetrazole, pyridinium trifluoroacetate and 4,5-dicyanoimidazole) were investigated with 2-cyanoethyl-*N,N,N',N'*-tetraisopropylphosphoramidite. 4,5-Dicyanoimidazole (DCI) gave the best results.

### EXAMPLE 2 - General experimental procedure for the preparation of LNA phosphoramidites 1-6:

A 0.2 M solution of the LNA nucleoside (1.0 equiv) in anhyd CH₂Cl₂ was stirred under argon. A 1.0 M solution of DCI (0.70 equiv) in anhyd MeCN was added followed by drop wise addition of *2-cyanoethyl-N,N,N',N'-tetraisopropylphosphorodiamidite* (1.0 equiv). When the reaction was completed (monitored by analytical TLC*) it was diluted with CH₂Cl₂ and washed twice with sat. aq NaHCO₃ and once with brine. The organic phase was dried (Na₂SO₄), filtered and concentrated *in vacou* to give a colourless foam. All phosphoramidites were isolated in very high yields (≥95%). No by-products could be detected by HPLC-MS, TLC or ³¹ P-NMR (purity≈100%). If starting materials are dry and pure chromatography is not necessary.
^{*} All phosphitylation reactions were completed in less than 2 h except for the G phosphoramidite **(2)** synthesis taking up to 4 h to be completed.

### Synthesis of (1R,3R,4R,7S)-3-(4-N-Benzoyl-5-methylcytosin-1-yl)-7-(2-cyanoethoxy-(diisopropylamino)phosphinoxy)-1-(4,4'-dimethoxytrityloxymethyl)-2,5-dioxabicyclo-[2.2.1]heptane (3)

(*1R,3R,4R,7S*)-3-(4-*N*-Benzoyl-5-methylcytosin-1-yl)-1-(4,4'-dimethoxytrityloxymethyl)-7-hydroxy-2,5-dioxabicyclo[2.2.1)heptane (4.86 g, 7.2 mmol) was dissolved in anhyd CH₂Cl₂ (40 mL) and 4,5-dicyanoimidazole in MeCN (1.0 M, 5 mL) was added. Stirred at ambient temperature under argon. 2-Cyanoethyl-*N,N,N',N'*-tetraisopropylphosphorodiamidite (2.4 ml, 7.2 mmol) was added drop wise to the reaction mixture. After 2 h the reaction was diluted with CH₂Cl₂ (100 mL) and transferred to a separation funnel and extracted with sat. aq. NaHCO₃ (2x150 mL) and brine (150 mL). The combined aqueous phases were extracted with CH₂Cl₂ (100 mL). The organic phases were pooled and dried (Na₂SO₄). After filtration the organic phase was evaporated *in vacou* to give a slightly yellow foam (6.0 g, 95% yield). A small sample was removed for comparison with the chromatographed material. The product was purified by Dry Column Vacuum Chromatography (⌀ 10 cm; Silica pretreated with 5% Et₃N in *n*-heptane v/v; 0→70% EtOAc, *n*-heptane v/v). Selected fractions containing 3 were combined and evaporated in *vacou* to give a colourless foam (5.3 g, 84% yield).

No difference in coupling efficiency could be detected on the oligonucleotide synthesiser in a coupling efficiency assay *(vide supra)* between the crude and chromatographed phosphoramidite.

The compounds **1**, **2**, **4**, **5** and **6** were prepared in a similar manner.

### Analytical data for phosphoramidites 1-6.

(*1R,3R,4R,7S)-7*-(2-Cyanoethoxy(diisopropylamino)phosphinoxy)-1-(4,4'-dimethoxytrityloxymethyi)-3-(thymin-1-yl)-2,5-dioxabicyclo[2:2:1]heptane **(1)**
³¹P-NMR (CDCl₃, 121.49 MHz): δ 150.0 (s), 149.3 (s).
MS (ES): *m*/*z* calcd for C₄₁H₄₉N₄O₉P [M+H]⁺: 773.3. Found: 773.1.
RP HPLC: R_{T} = 5.89 min, 6.19 min.

(*1R,3R,4R,7S)*-7-(2-Cyanoethoxy(diisopropylamino)phosphinoxy)-1-(4,4'-dimethoxytrityloxymethyl)-3-(2-*N*-isobutyrylguanin-1-yl)-2,5-dioxabicyclo[2.2.1]heptane **(2)**
³¹P-NMR (CDCl₃, 121.49 MHz): δ 150.2 (s), 149.2 (s).
MS (ES): m/z calcd for C₄₅H₅₄N₇O₉P [M+H]⁺: 868.4. Found: 868.0.
RP HPLC: R_{T} = 5.52 min, 5.72 min.

(*1R,3R,4R,7S)-*3-(4-*N*-Benzoyl-5-methylcytosin-1-yl)-7-(2-cyanoethoxy(diisopropylamino)phosphinoxy)-1-(4,4'-dimethoxytrityloxymethyl)-2,5-dioxabicyclo[2.2.1]heptane **(3)**
³¹P-NMR (CDCl₃, 121.49 MHz): δ 150.5 (s), 150.5 (s).
MS (ES): *m*/*z* calcd for C₄₈H₅₄N₅O₉P [M+H]⁺: 876.4. Found: 876.2.
RP HPLC: R_{T} = 7.67 min, 8.13 min.
(HPLC solvent gradient: 0.0-0.5 min 95% B, 0.5-2.0 min 95%→100% B, 2.0-7.0 min 100% B, 7.0-7.5 min 100%→95% B, 7.5-12.0 min 95% B)

*(1R,3R,4R,*7S)-3-(6-*N*-Benzoyladenin-9-yl)-7-(2-cyanoethoxy(diisopropylamino)-phosphinoxy)-1-(4,4'-dimethoxytrityloxymethyl)-2,5-dioxabicyclo[2.2.1]heptane **(4)**
³¹P-NMR (CDCl₃, 121.49 MHz): δ 150.1 (s), 149.7 (s).
MS (ES): *m*/*z* calcd for C₄₈H₅₂N₇O₈P [M+H]⁺: 886.3. Found: 886.0.
RP HPLC: R_{T} = 6.65 min, 6.82 min.

*(1R,3R,4R,* 7S)-7-(2-Cyanoethoxy(diisopropylamino)phosphinoxy)-1-(4,4'-dimethoxytrityloxymethyl)-5-*N*-methyl-3-(thymin-1-yl)-2-oxa-5-azabicyclo[2.2.1]heptane **(5)**
³¹P-NMR (CDCl₃, 121.49 MHz): **δ** 149.8 (s), 149.6 (s).
MS (ES): *m*/*z* calcd for C₄₂H₅₂N₅O₈P [M+H]⁺: 786.3. Found: 786.2.
RP HPLC: R_{T} = 5.87 min, 6.26 min.

*(1S,3R,4S,7R*)-7-(2-Cyanoethoxy(diisopropylamino)phosphinoxy)-1-(4,4'-dimethoxytrityloxymethyl)-3-(thymin-1-yl)-2,5-dioxabicyclo[2:2:1]heptane **(6)**
³¹P-NMR (CDCl₃, 121.49 MHz): δ 150.9 (s), 150.6 (s).
MS (ES): *m*/*z* calcd for C₄₁H₄₉N₄O₉P [M+H]⁺: 773.3. Found: 773.1.
RP HPLC: R_{T} = 6.06 min, 6.44 min.

## Claims

1. A method for the synthesis of an LNA phosphoramidite, the method comprising phosphitylation of the 3'-OH group of an LNA monomer with a 2-cyanoethyl-*N,N, N', N'-* tetra-substituted phosphoramidite in the presence of a nucleophilic activator selected from 4,5-dicyanoimidazole (DCI), 4-alkylthioimidazole, 2-alkylthioimidazole, 2-nitroimidazole, 4-nitroimidazole, 4,5-dihaloimidazole, 4-haloimidazole, 2-haloimidazole and 5-alkoxytetrazole.

2. The method according to claim 1, which comprises at the most one precipitation step.

3. The method according to claim 1 or 2, wherein the 2-cyanoethyl-*N,N,N*',*N'*-tetra-substituted phosphoramidite is selected from 2-cyanoethyl-*N,N,N',N'*-tetraisopropylphosphoramidite and 2-cyanoethyl-*N,N,N',N'*-tetraethylphosphoramidite.

4. The method according to claims 1 or 2, wherein the 2-cyanoethyl-*N,N,N'N'-*tetra-substituted phosphoramidite is 2-cyanoethyl-*N,N,N'N'*-tetraisopropylphosphoramidite and the nucleophilic activator is 4,5-dicyanoimidazole.

5. The method according to any of the preceding claims, wherein the molar ratio between the LNA monomer and the nucleophilic activator is in the range of 1:0.0001 to 1:10.

6. The method according to any of the preceding claims, wherein the molar ratio between LNA monomer and the 2-cyanoethyl-*N,N,N,'N'*-tetra-substituted phosphoramidite PN₂-reagent is in the range of 1:0.9 to 1:10.

7. The method according to any of the preceding claims, wherein the phosphitylation is allowed to proceed for 0.2-8 hours.

8. The method according to any of the preceding claims, wherein the LNA monomer has a nucleobase selected from guanine; thymine, cytosine, 5-methylcytosine uracil and adenine.

9. The method according to any of the preceding claims, wherein the LNA monomer structure is selected from oxy β-D-ribo-LNA, thio-β-D-ribo-LNA, amino-β-D-ribo-LNA, oxy-α-L-ribo-LNA, thio-α-L-ribo-LNA and amino-α-L-ribo-LNA.

## Patentansprüche

1. Verfahren zur Herstellung eines LNA Phosphoramidites, wobei das Verfahren eine Phosphitylierung der 3'-OH Gruppe eines LNA Monomers mit einem 2-Cyanoethyl-N,N,N',N'-tetra-substituierten Phosphoramidit in Gegenwart eines nukleophifen Aktivators ausgewählt aus 4,5-Dicyanoimidazol (DCI), 4-Alkylthioimidazol, 2-Alkylthioimidazol, 2-Nitroimidazol, 4-Nitroimidazol, 4,5-Dihaloimidazol, 4-Haloimidazol, 2-Haloimidazol und 5-Alkoxytetrazol beinhaltet.

2. Verfahren nach Anspruch 1, umfassend wenigstens einen Fällungsschritt.

3. Verfahren nach Anspruch 1 oder 2, bei dem das 2-Cyanoethyl-N,N,N',N'tetra-substituierte Phosphoramidit ausgewählt wird aus 2-Cyanoethyl-N,N,N',N'-Tetraisopropylphosphoramidit und 2-Cyanoethyl-N,N,N',N'-Tetraethylphosphoramidit.

4. Verfahren nach den Ansprüchen 1 oder 2, bei dem das 2-Cyanoethyl-N,N,N',N'-tetra-substituierte Phosphoramidit 2-Cyanoethyl-N,N,N',N'-Tetraisopropylphosphoramidit und der nukleophile Aktivator 4,5-Dicyanoimidazol ist.

5. Verfahren nach einem der vorhergehenden Ansprüche, bei dem das molare Verhältnis zwischen dem LNA Monomer und dem nukleophilen Aktivator im Bereich von 1:0,0001 bis 1:10 liegt.

6. Verfahren nach einem der vorhergehenden Ansprüche, bei dem das molare Verhältnis zwischen dem LNA Monomer und dem 2-Cyanoethyl-N,N,N',N'-tetrasubstituierten Phosphoramidit PN₂-Reagenz im Bereich von 1:0,9 bis 1:10 liegt.

7. Verfahren nach einem der vorhergehenden Ansprüche, bei dem die Phosphitylierung für 0,2 bis 8 Stunden aufrechterhalten wird.

8. Verfahren nach einem der vorhergehenden Ansprüche, bei dem das LNA Monomer eine Nukleinbase ausgewählt aus Guanin, Thymin, Cytosin, 5-Methylcytosin, Uracil und Adenin aufweist.

9. Verfahren nach einem der vorhergehenden Ansprüche, bei dem die Struktur des LNA Monomers ausgewählt ist aus oxy-β-D-Ribo-LNA, thio-β-D-Ribo-LNA, amino-β-D-Ribo-LNA, oxy-α-L-Ribo-LNA, thio-α-L-Ribo-LNA und amino-α-L-Ribo-LNA.

## Revendications

1. Procédé de synthèse d'un phosphoramidite d'acide nucléique verrouillé (LNA), le procédé comprenant la phosphitylation du groupe 3'-OH d'un monomère d'acide nucléique verrouillé avec un phosphoramidite à substitution *2-cyanoéthyl-N,N,N',N'-tétra* en présence d'un activateur nucléophile choisi parmi le 4,5-dicyanoimidazole (DCI), le 4-alkylthioimidazole, le 2-alkylthioimidazole, le 2-nitroimidazole, le 4-nitroimidazole, le 4,5-dihaloimidazole, le 4-haloimidazole, le 2-haloimidazole et le 5-alcoxytétrazole.

2. Procédé selon la revendication 1, lequel comprend tout au plus une étape de précipitation.

3. Procédé selon la revendication 1 ou 2, dans lequel le phosphoramidite à substitution 2-cyanoéthyl-*N,N,N',N'*-tétra est choisi parmi le 2-cyanoéthyl-*N,N,N',N'*-tétraisopropylphosphoramidite et le 2-cyanoéthyl-*N,N,N',N'*-tétraéthylphosphoramidite.

4. Procédé selon les revendications 1 ou 2, dans lequel le phosphoramidite à substitution 2-cyanoéthyl-*N,N,N',N'*-tétra est le 2-cyanoéthyl-*N,N,N',N'*-tétraisopropylphosphoramidite et l'activateur nucléophile est le 4,5-dicyanoimidazole.

5. Procédé selon l'une quelconque des revendications précédentes, dans lequel le rapport molaire entre le monomère d'acide nucléique verrouillé et l'activateur nucléique est dans la plage de 1/0,0001 à 1/10.

6. Procédé selon l'une quelconque des revendications précédentes, dans lequel le rapport molaire entre le monomère d'acide nucléique verrouillé et le réactif PN₂ du phosphoramidite à substitution 2-cyanoéthyl-*N,N,N',N'*-tétra est dans la plage de 1/0,9 à 1/10.

7. Procédé selon l'une quelconque des revendications précédentes, dans lequel on laisse la phosphitylation se poursuivre pendant 0,2 à 8 heures.

8. Procédé selon l'une quelconque des revendications précédentes, dans lequel le monomère d'acide nucléique verrouillé a une nucléobase choisie parmi la guanine, la thymine, la cytosine, la 5-méthylcytosine, l'uracile et l'adénine.

9. Procédé selon l'une quelconque des revendications précédentes, dans lequel la structure du monomère d'acide nucléique verrouillé est choisie parmi oxy-β-D-ribo-LNA, thio- β -D-ribo-LNA, amino- β -D-ribo-LNA, oxy- α -L-ribo-LNA, thio-α -L-ribo-LNA et amino- α -L-ribo-LNA.
